# EUROPEAN PATENT APPLICATION

(11) **EP 2 474 294 A1**
(43) Date of publication of application: **11.07.2012**
(21) Application number: 10813744.9
(22) Date of filing: 01.09.2010
(51) Int. Cl.: A61J 3/07, A61J 1/03, A61J 1/05, A61J 7/00, A61K 9/14, A61K 9/20

(54) **PREPARATION FOR ORAL ADMINISTRATION**

(30) Priority: 02.09.2009 JP 2009202760
(71) Applicant: Morimoto-Pharma Co., Ltd., Suita-shi, Osaka 565-0843 (JP)
(72) Inventor: MORIMOTO Shuji, Suita-shi Osaka 565-0843 (JP); SAKUMA Yutaka, Suita-shi Osaka 565-0843 (JP)
(74) Representative: Fleuchaus, Michael A.
(86) International application number: PCT/JP2010/064957
(87) International publication number: WO 2011/027795

(57) **Abstract**

Disclosed is a preparation for oral administration which can reduce the residual amount of a medicinal composition and facilitate the swallowing of the medicinal composition. A preparation (10) for oral administration comprises an enclosing member (20) that is formed of an edible film. A medicinal composition (50) is enclosed in the enclosing member (20). The enclosing member (20) has a wall part (40). The wall part (40) is formed by rolling the edible film into a tubular shape. The wall part (40) has a part (60) where the edible film overlaps. The part (60) where the edible film overlaps is formed by rolling a single edible film sheet.

## Description

### Technical Field

This invention relates to a preparation for oral administration and to a production method for a preparation for oral administration, more specifically, to a preparation for oral administration and a production method of a preparation for oral administration allowing an improvement of productivity.

### Background Art

Orally administered solid preparations include relatively large ones such as pills, tablets or capsules, and finely granulated ones such as powder or granular preparations. Generally, such preparations are all swallowed using water or the like. However, particularly aged people, infants, weak and sick people and so forth have the problem that it is difficult to swallow large solid preparations such as tablets. Meanwhile, particularly in the case of aged people, infants, weak and sick people and so forth, fine powder preparations sometimes spread inside the entire mouth or adhere to the throat, with a portion of the preparation remaining for a long time; therefore there is the problem of those persons feeling a strong resistance towards taking the preparation. The reason for the strong resistance is that because the powder is temporarily received on the tongue, bitterness and the like of the drug ingredients are felt intensely. Moreover, while lying down, it is difficult to ingest a solid preparation with water, which is highly fluid. Therefore it is necessary to make people sit up each time when taking medicine. In some cases, this is a large burden to the patient.

Patent document 1 discloses a drug preparation package. Said drug preparation package comprises a bag-like storage member and a drug preparation stored in the bag-like storage member. The bag-like storage member comprises a circumferential seal section. The seal section is formed by overlaying and sealing edible films.

According to the drug preparation package disclosed in patent document 1, the possibility of choking or sensing a strong bitterness can be reduced, the drug preparation can be taken with less discomfort in the mouth, no complicated operation is required to take the drug preparation, and it is possible to easily control whether or not the required quantity was taken.

### Prior Art Documents

### Patent Documents

Patent document 1: Japanese Patent Laid-Open No. 2009-61108

### Brief Summary of the Invention

### Problem to Be Solved By the Invention

However, the invention disclosed in patent document 1 exhibits the problem that there is room for improvement of the productivity. According to patent document 1, the drug preparation package is made by overlaying edible films, heating and compressing the circumference thereof to form a bag-like storage member, and storing a predetermined quantity of a drug preparation inside the bag-like storage member. That there is room for improvement in the productivity is because the work of storing the drug preparation in the bag-like storage member tends to be time-consuming.

This invention was made to solve the abovementioned problem, and the purpose of the invention is to provide a preparation for oral administration that allows an improvement of productivity.

### Means for Solving the Problem

The preparation for oral administration of this invention is explained in reference to the drawings. Note that the use of the reference numerals of the drawings in this column is intended to facilitate the understanding of the content of the invention and is not intended to limit the content to the scope indicated.

According to the aspects of this invention to achieve the abovementioned purpose, the preparations 10 and 70 for oral administration are provided with enclosing members 20 and 92, which are made of edible film. The enclosing members 20 and 92 contain a medicinal composition 50. The enclosing members 20 and 92 comprise a wall part 40. The wall part 40 is formed by rolling an edible film into a tubular shape.

Since the wall part 40 is formed by rolling an edible film into a tubular shape, the wall part 40 has a tubular shape. Since the wall part 40 has a tubular shape, the space for filling in the medicinal composition 50 is already formed at the time when the wall part 40 is formed. Since the space for filling in the medicinal composition 50 is already formed, the medicinal composition 50 can easily be filled in. Since the medicinal composition 50 can easily be filled in, the productivity can be improved.

Further, it is preferable that the abovementioned wall part 40 comprise a portion 60 where the edible film overlaps. It is not easy to freely control the thickness of an edible film. Since the portion 60 where the edible film overlaps is comprised by the wall part 40, it becomes possible to control the thickness of the wall part 40 in said portion freely to a certain extent by controlling how many layers of the edible film are overlaid in the portion 60 where the edible film overlaps. As a result, the productivity can be improved in cases where it is necessary to control the thickness of the wall part 40.

Otherwise, it is preferable that the abovementioned portion 60 where the edible film overlaps be formed by rolling one sheet of edible film. The work of forming the portion 60 where the edible film overlaps by rolling one sheet of edible film can be executed extremely quickly. As a result, it is possible to improve the productivity for the forming of the wall part 40.

Moreover, it is preferable that the end of the abovementioned edible film be adhered to the wall part 40. By the adhesion of the end of the edible film to the wall part 40, the outer circumference of the wall part 40 will become closed.

Further, it is preferable that the abovementioned preparation 10 for oral administration additionally comprise a package bag 22. In such case, the enclosing member 20 is stored in the package bag 22.

Moreover, it is preferable that the abovementioned enclosing member 20 additionally comprise a bottom section 42 and a mouth section 44. The bottom section 42 is disposed on one end of the wall part 40. The mouth section 44 is disposed on the other end of the wall part 40 and is closed.

Further, it is preferable that the abovementioned preparation 70 for oral administration comprise a drug container 72 in addition to the enclosing member 92. The drug container 72 comprises a plurality of spaces 80, 82, 84 and 86. In this case, the portions between two adjoining spaces are closed. The portions between two adjoining spaces open when force is applied from the outside of the drug container 72. A predetermined aperture part 160 is disposed on the drug container 72. The predetermined aperture part 160 is predetermined for forming an aperture which lets the outside of the drug container 72 communicate with the spaces 80, 82, 84 and 86. A swallowing aid substance 90 is stored in a swallowing-aid substance storage chamber 86, which is at least one of the spaces 80, 82, 84 and 86. The enclosing member 92 is stored in an enclosing-member storage chamber 82, which is at least one of the spaces 80, 82, 84 and 86.

Otherwise, it is preferable that the abovementioned enclosing member 92 be stored in the enclosing-member storage chamber 82 in such a state that the wall part 40 is bent.

Otherwise, it is preferable that the abovementioned bent state of the wall part 40 be a state of being bent to a curl shape.

Otherwise, it is preferable that both ends of the abovementioned enclosing member 92 be oriented towards the swallowing-aid substance storage chamber 86.

According to the other aspects of this invention, the preparation for oral administration comprises an enclosing member 200 made of edible film. The enclosing member 200 contains a medicinal composition 50. The enclosing member 200 comprises a wall part 240. The wall part 240 is formed by rolling an edible film into a tubular shape. The wall part 240 comprises a portion 60 where the edible film overlaps. The medicinal composition 50 contains at least 99 weight percent of particles with a particle size exceeding 100 micrometers.

According to the other aspects of this invention, the production method for the preparation 10 for oral administration comprises a wall forming step and a medicinal composition filling step. The preparation 10 for oral administration comprises an enclosing member 20 made of edible film. The enclosing member 20 contains a medicinal composition 50. The enclosing member 20 comprises a wall part 40. The wall part 40 consists of edible film. In the wall forming step, a tubular shaped wall part 40 is formed by rolling an edible film. In the medicinal composition filling step, the medicinal composition 50 is filled from one end of the wall part 40 into the space enclosed by the wall part 40.

Moreover, it is preferable that the abovementioned enclosing member 20 additionally comprise a bottom section 42 and a mouth section 44. In this case, the bottom section 42 is disposed on one end of the wall part 40. The mouth section 44 is disposed on the other end of the wall part 40 and is closed. The production method of the preparation 10 for oral administration further comprises a bottom forming step and a closing step. In the bottom forming step, the bottom section 42 is formed by compressing and fusion-bonding one end of the wall part 40. In the closing step, the mouth section 44 is closed by compressing and fusion-bonding the other end of the wall part 40 after the medicinal composition filling step.

### Effect of the Invention

According to this invention, it is possible to improve productivity.

### Brief Description of the Drawings

FIG. 1 is a partial cross section of the preparation for oral administration of example 1 of this invention.
FIG. 2 is a partial cross section of the enclosing member of example 1 of this invention.
FIG. 3 is a conceptual diagram showing the production steps of the preparation for oral administration of example 1 of this invention.
FIG. 4 is a partial cross section of the preparation for oral administration of example 2 of this invention.
FIG. 5 is a conceptual diagram showing the production steps of the preparation for oral administration of example 2 of this invention.
FIG. 6 is a conceptual diagram showing the content of the enclosing-member filling step of example 2 of this invention.
FIG. 7 is a partial cross section of the preparation for oral administration of example 3 of this invention.
FIG. 8 shows the results of the leakage test of example 3 of this invention.

### Modes for Carrying Out the Invention

The following explains the examples of this invention on the basis of the drawings. In the following explanations, identical parts are assigned the same reference numeral. The names and functions thereof are also the same. Accordingly, detailed explanations thereof will not be repeated.

### <Example 1>

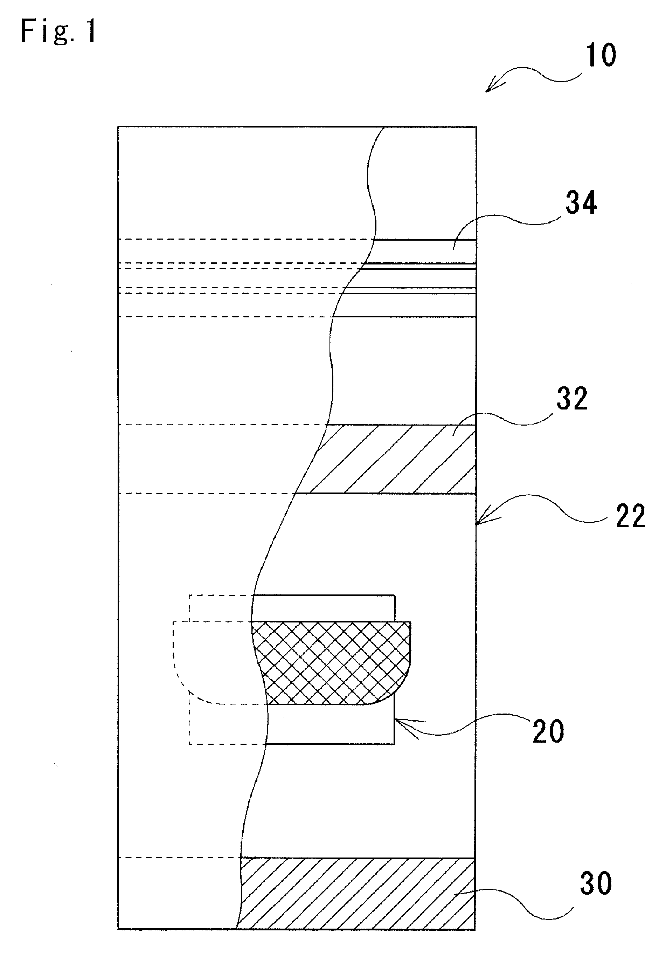

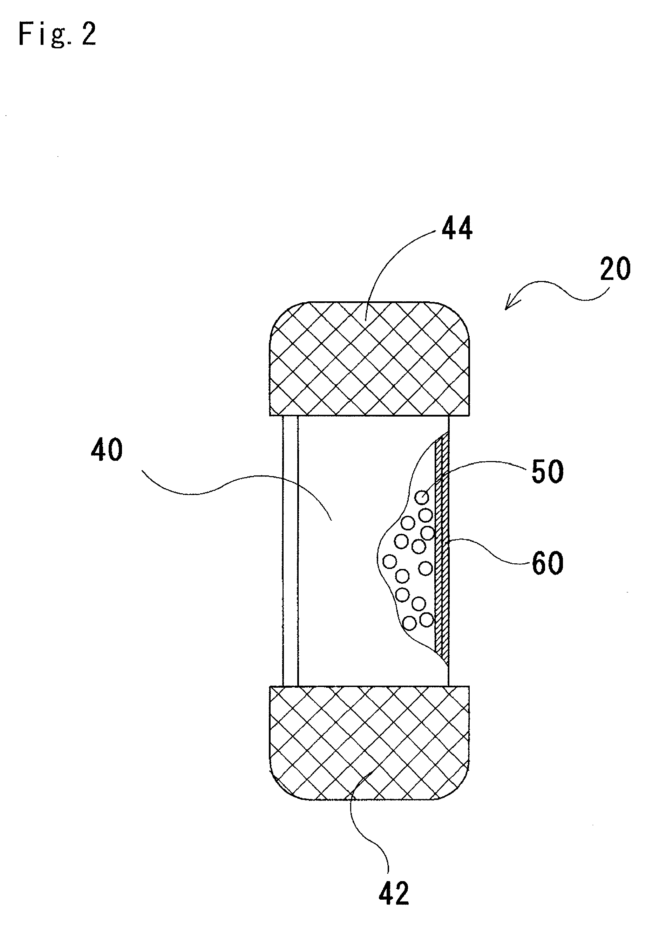

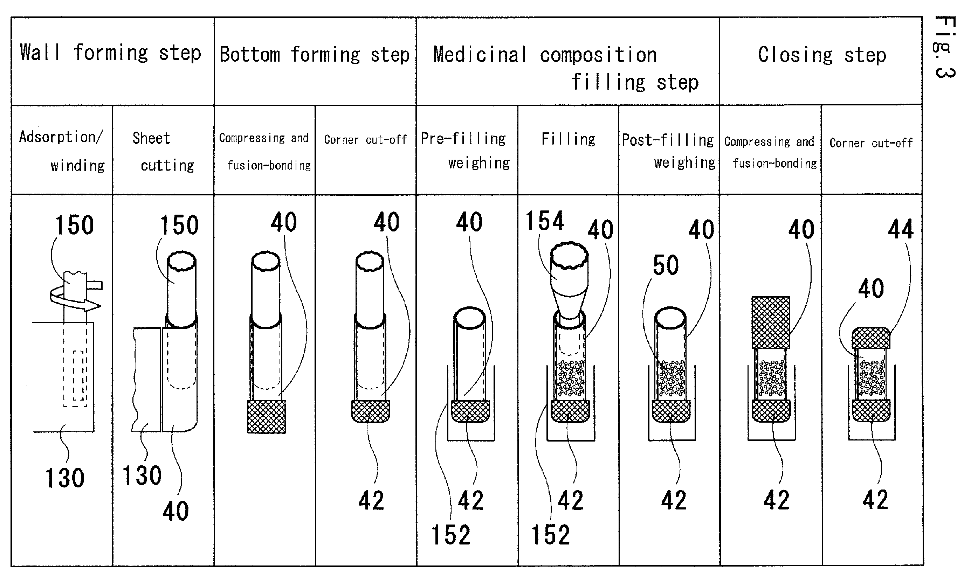

FIG. 1 is a partial cross section of the preparation for oral administration of this example. FIG. 2 is a partial cross section of the enclosing member 20 of this example. FIG. 3 is a conceptual diagram showing the production steps of the preparation for oral administration of this example.

The preparation for oral administration of this example stores the enclosing member 20 in the package bag 22. The package bag 22 is formed by fusion-bonding the bottom of a tubular shaped vinyl (the fusion-bonded portion is the package-bag bottom section 30). Further, the package bag 22 comprises a weak seal section 32 and a sealing zipper 34. The weak seal section 32 is a portion that is fusion-bonded more weakly than the package-bag bottom section 30. The weak seal section 32 easily opens when the outside of the package bag 22 is held with both hands and a pulling force is applied to the weak seal section 32. The sealing zipper 34 is a widely-known zipper provided for preventing air from entering and exiting. Accordingly, a detailed explanation thereof will not be repeated here.

The enclosing member 20 contains a medicinal composition 50 which is a granular drug or a powdered drug. The enclosing member 20 comprises a wall part 40, a bottom section 42 and a mouth section 44. In this example, the wall part 40 is made of two oblate (an example for an edible film) layers 60 that are overlaid. The layers are overlaid by rolling one oblate sheet into a tubular shape. The bottom section 42 is formed by fusion-bonding one end of the wall part 40. The same applies for the mouth section 44. Note that this example uses an oblate that is made of starch and has a thickness of 10 µm. In case it is necessary to control the time from when the swallowing aid substance 90 covers the surface of the enclosing member 92 until the enclosing member 92 completely melts, the thickness of the oblate and the number of oblate layers 60 should be selected as appropriate.

The production method of the preparation for oral administration of this example will now be explained. The production method of the preparation for oral administration of this example comprises an enclosing-member production step and an enclosing-member storing step. The enclosing-member production step is for producing the enclosing member 20. The enclosing-member storing step is for storing the enclosing member 20 in the package bag 22.

As is shown in FIG. 3, the enclosing-member production step comprises a wall forming step, a bottom forming step, a medicinal composition filling step and a closing step. In the wall forming step, a tubular shaped wall part 40 is formed by rolling an oblate. In the bottom forming step, the bottom section 42 is formed by compressing one end of the oblate tube formed in the wall forming step and closing the compressed end by fusion-bonding. In the medicinal composition filling step, the medicinal composition is filled into the wall part 40. In the closing step, the mouth section 44 is formed by fusion-bonding (compressing) the other end of the oblate tube that passed through the medicinal composition filling step to close same.

The wall forming step comprises an adsorption and winding step and a sheet cutting step. In the adsorption and winding step, the sheet-like oblate 130 is adsorbed to an adsorption tube 150, which is equipped with a hole on the tip (the adsorption tube 150 can rotate with the center axis as the rotation axis), and wound to the outer circumference thereof. Since the adsorption tube 150 can rotate, the oblate 130 can easily be wound to the outer circumference of the adsorption tube 150 without particularly moving the oblate 130. In the sheet cutting step, the oblate 130 is cut off after the oblate 130 is wound to the outer circumference of the adsorption tube 150.

The bottom forming step comprises a compressing and fusion-bonding step, and a corner cut-off step. In the compressing and fusion-bonding step, one end of the tubular shaped oblate formed in the adsorption and winding step is compressed and the compressed end is closed by fusion-bonding. In the corner cut-off step, the corners of the oblate end that is closed by fusion-bonding are cut off so as to be rounded. Thereby, the bottom section 42 is completed.

The medicinal composition filling step comprises a pre-filling weighing step, a filling step and a post-filling weighing step. In the pre-filling weighing step, the mass of the oblate tube, the bottom section 42 of which was completed in the bottom forming step, is measured. In the pre-filling weighing step, the oblate tube is first inserted into the edible film container 152. The insertion of the oblate tube into the edible film container 152 is performed by first inserting the oblate tube together with the adsorption tube 150 into the edible film container 152, and then releasing air from the adsorption tube 150 to pull out the adsorption tube 150 while leaving only the oblate tube in the edible film container 152. In the filling step, the medicinal composition is filled by an auger filling machine 154 into the wall part 40. In the post-filling weighing step, the mass of the oblate tube into which the medicinal composition was filled during the filling step is measured together with the medicinal composition. Note that the difference between the mass of the oblate tube in the post-filling weighing step and the mass of the oblate tube in the pre-filling weighing step not being within a predetermined range means that the filling quantity of the medicinal composition 50 is not within the predetermined range. When the difference between the mass of the oblate tube in the post-filling weighing step and the mass of the oblate tube in the pre-filling weighing step is not within a predetermined range, the oblate tube of which the mass was measured is abandoned. The medicinal composition 50 therein is recovered.

Same as the bottom forming step, the closing step also comprises a compressing and fusion-bonding step, and a corner cut-off step. In the compressing step of the closing step, the other end of the tubular shaped oblate into which the medicinal composition was filled is compressed and the said compressed other end is closed by fusion-bonding. The compressing step and the fusion-bonding step may be performed either simultaneously or sequentially. In the corner cut-off step, the corners of the other oblate end that was closed by fusion-bonding are cut off so as to become rounded. Thereby, the mouth section 44 is completed. Consequently, the enclosing member 20 is completed.

Next, it will be explained how to ingest the preparation for oral administration of this example while referring to FIG. 1. The person intending to ingest the preparation first picks up the outer circumference of the package bag 22 and pulls same to tear open the weak seal section 32. When the weak seal section 32 is open, the person opens the sealing zipper 34 and takes out the enclosing member 20. Once the enclosing member 20 is taken out, the person places the enclosing member 20 into his or her mouth and immediately swallows the enclosing member 20 together with a sufficient quantity of water. By including water to a sufficient degree, the surface of the enclosing member 20 melts and becomes extremely slippery inside the mouth.

In the abovementioned manner, the enclosing member formed by rolling an edible film (an oblate in this example) into a tubular shape is used for the preparation for oral administration and the production method thereof in this example. Since said enclosing member is used, the mouth of the edible film is open when filling in the medicinal composition. Since the mouth is open, the mouth of the auger filling machine 154 (there are various shapes for the filling mouth and various filling methods for filling in the medicinal composition 50. It should be unnecessary to say that the use of an auger filling machine 154 in this example is merely one in many examples.) can easily enter same. Since the mouth of the auger filling machine 154 can easily enter, the efficiency of the medicinal composition filling step improves. Consequently, the efficiency of the production of the preparation for oral administration improves.

Further, the production method of the preparation for oral administration of this example, that is to say, the filling method of the medicinal composition of this example comprises a step in which, at first, the mass of the edible film (an oblate in this example) which was rolled into a tubular shape is measured, a step in which the medicinal composition 50 is filled thereinto using an auger filling machine 154, and a step in which, after the filling, the mass of the edible film is measured. If the results from measuring the mass of the edible film before and after the filling reveal that the difference thereof, in other words, the net filling quantity, is not within a predetermined range, the medicinal composition 50 inside the edible film is recovered. The edible film from which the medicinal composition 50 was recovered is abandoned. If the mass difference is within the predetermined range, the edible film becomes the enclosing member 20, and if the mass difference is not within the predetermined range, the medicinal composition 50 is recovered; therefore it is possible to keep variations of the mass of the medicinal composition in the produced enclosing member 20 small. Furthermore, it is possible to increase the substantial yield of the medicinal composition 50. Moreover, when using the auger filling machine 154, it is possible to compare the abovementioned net filling quantity with the target filling quantity and adjust the set rotation angle (that is to say, the feeding speed of the medicinal composition 50) of the auger filling machine 154 according to the difference thereof. Consequently, when using the auger filling machine 154, it is possible to easily control the feeding quantity of the medicinal composition 50 so as to become the target filling quantity. In this respect, it is preferable that the auger filling machine 154 be used.

Moreover, according to the preparation 10 for oral administration of this example, the thickness of the wall part 40 of the enclosing member 20 can easily be changed by a change of the number of edible film layers. The thickness of the wall part 40 affects the elution speed of the medicinal composition 50 from the enclosing member 20; therefore being easily able to change the thickness of the wall part 40 means that the elution properties of the medicinal composition 50 being released from the enclosing member 20 can easily be controlled. As a result, it is possible to provide an enclosing member 20 which allows an easy control of the elution properties of the medicinal composition 50 which is released therefrom.

### <Example 2>

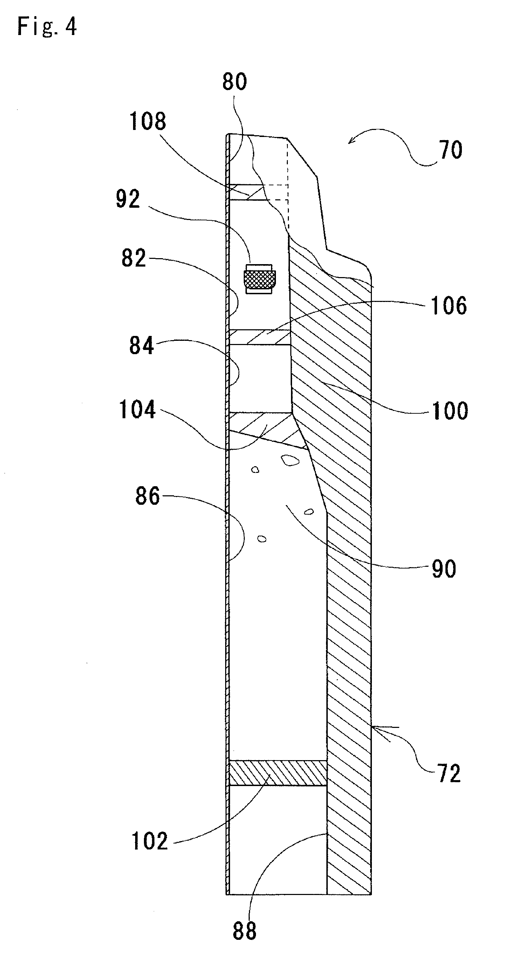

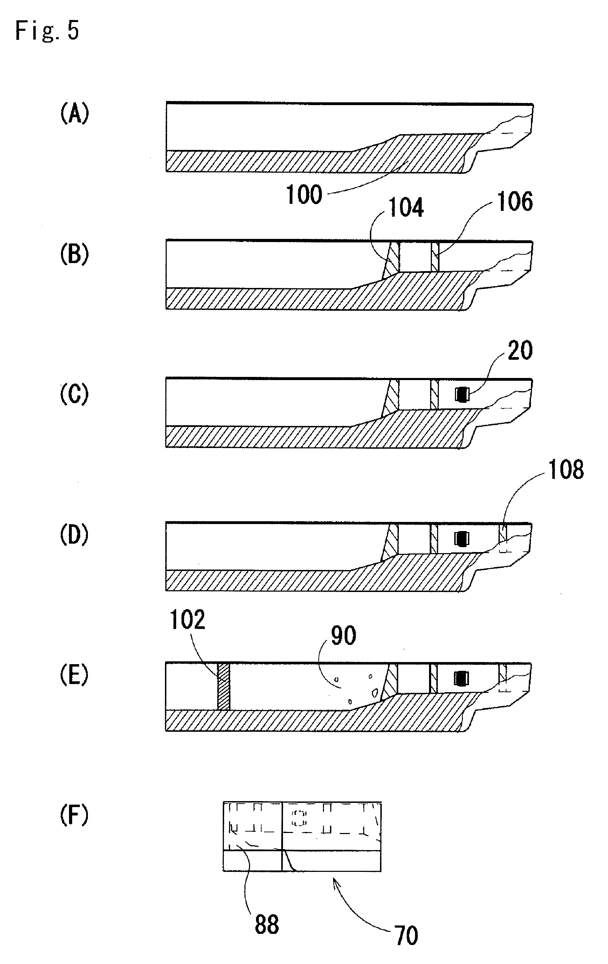

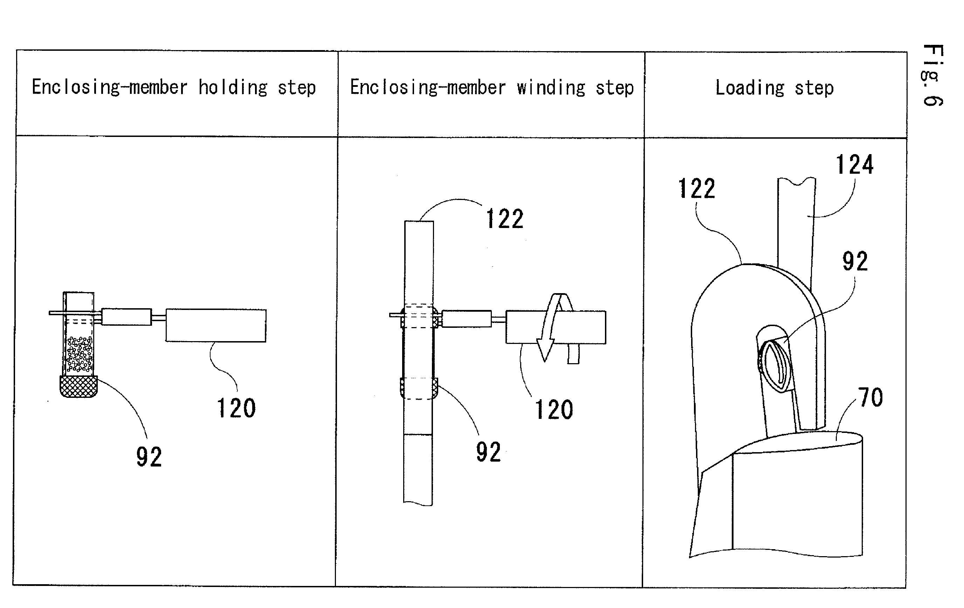

FIG. 4 is a partial cross section of the preparation 70 for oral administration of this example. FIG. 5 is a conceptual diagram showing the production steps of the preparation 70 for oral administration of this example. FIG. 6 is a conceptual diagram showing the content of the enclosing-member filling step of this example.

The preparation 70 for oral administration of this example is formed by bonding the circumference of a double-folded synthetic resin (preferably a soft synthetic resin that can be folded, such as low-density polyethylene) sheet. The preparation 70 for oral administration has a swallowing-aid substance storage chamber 86, an intermediate chamber 84, an enclosing-member storage chamber 82 and an outlet chamber 80 formed therein. The swallowing-aid substance storage chamber 86, the intermediate chamber 84 and the enclosing-member storage chamber 82 are so formed as to maintain air tightness with respect to the external space. The swallowing-aid substance storage chamber 86, the intermediate chamber 84, the enclosing-member storage chamber 82 and the outlet chamber 80 are arranged in one row.

A swallowing aid substance 90 is stored inside the swallowing-aid substance storage chamber 86. The swallowing aid substance 90 in this example is a jelly. The outlet chamber 80 in this example is an empty chamber until the third weak seal portion 108, which will be explained later in this example, is broken.

An enclosing member 92 is stored inside the enclosing-member storage chamber 82. The enclosing member 92 of this example is made by bending a half-finished product of the enclosing member 20 of the example 1 wherein the mouth section 44 is not yet formed, to a curl shape. Therefore, a detailed explanation thereof will not be repeated here.

Note that, if necessary, a gas (for example, nitrogen gas) which does not have any effect on the medicinal composition 50 or the swallowing aid substance 90 is filled into the enclosing-member storage chamber 82, the intermediate chamber 84 and the swallowing-aid substance storage chamber 86.

The portion between the swallowing-aid substance storage chamber 86 and the intermediate chamber 84 is partitioned by a first weak seal portion 104. The portion between the intermediate chamber 84 and the enclosing-member storage chamber 82 is partitioned by a second weak seal portion 106. The portion between the enclosing-member storage chamber 82 and the outlet chamber 80 is partitioned by a third weak seal portion 108. The first weak seal portion 104 to third weak seal portion 108 are those parts of the portion where the sheet forming the preparation 70 for oral administration is bonded, which correspond to the portions between two adjoining spaces. The strength of the first weak seal 104 to third weak seal 108 is weaker than the strength of the circumferential strong seal portion 100. Configuring the strength of the bonded portions differently becomes possible by setting an appropriate pressure, temperature and time when bonding by heat-sealing. Even with other sealing methods, for example, ultrasonic sealing or impulse sealing, it becomes possible to adjust the strength of the bonded portions by adjusting the sealing time or strength as appropriate. In addition, it is possible to form the first weak seal portion 104 to third weak seal portion 108 by adhering a film with a different melting temperature or by applying an adhesive agent to the inner surface of the sheet. As a result, the first weak seal portion 104 to third weak seal portion 108 are easily broken by the force applied by the swallowing aid substance 90 to the swallowing-aid substance storage chamber 86 or the enclosing-member storage chamber 82 when the swallowing-aid substance storage chamber 86 or the enclosing-member storage chamber 82 etc. are pressed from the outside of the preparation 70 for oral administration. That is to say, the portion between the swallowing-aid substance storage chamber 86 and the enclosing-member storage chamber 82 is sealed in such a state that the swallowing-aid substance storage chamber 86 and the enclosing-member storage chamber 82 can be interconnected with each other by means of force applied from the outside of the preparation 70 for oral administration.

A cover section 88 is disposed on the portion corresponding to the opposite side of the outlet chamber 80 of the preparation 70 for oral administration. The portion between the swallowing-aid substance storage chamber 86 and the cover section 88 is partitioned by an inner strong seal section 102. The strength of the inner strong seal section 102 is roughly the same as the strength of the circumferential strong seal portion 100.

Next, it will be explained how to produce the preparation 70 for oral administration of this example while referring to FIG. 5 and FIG. 6. First, a sheet is double-folded. The ends of the double-folded sheet are fusion-bonded by application of heat for a predetermined time and at a predetermined pressure and temperature according to preset conditions for the circumferential strong seal portion 100. The ends of the fusion-bonded sheet are cut to form the outer shape of the preparation 70 for oral administration. FIG. 5(A) shows such state.

Once the outer shape of the preparation 70 for oral administration is formed, heat is applied from the outside to the portion where the first weak seal portion 104 is to be formed and to the portion where the second weak seal portion 106 is to be formed, for a predetermined time and at a predetermined pressure and temperature according to preset conditions for the first weak seal portion 104 and the second weak seal portion 106. Thereby, the inner surface of the sheet is fusion-bonded to form the first weak seal portion 104 and the second weak seal portion 106. FIG. 5 (B) shows such state.

Once the first weak seal portion 104 and the second weak seal portion 106 are formed, the enclosing member 92 is inserted into the double-folded sheet. The specific procedure therefor will be explained while referring to FIG. 6.

First, the end (the end with the open mouth) of the enclosing member 92 is held by a holding jig 120. The "enclosing-member holding step" of FIG. 6 shows such state. Note that, as mentioned above, the enclosing member 92 of this example is a half-finished product (wherein the mouth section 44 is not yet formed) of the enclosing member 20 of example 1. Accordingly, a detailed explanation of the production method for the enclosing member 92 will not be repeated here.

Once the end of the enclosing member 92 is held, the holding jig 120 is rotated to thereby wind the enclosing member 92 to the holding jig 120. When doing so, the holding jig 120 is rotated while the enclosing member 92 is pressed against the hook-like portion of the loading jig 122. Thereby it is possible to wind the enclosing member 92 in a compact manner. As a side note, since one end of the wall part 40 is open, the possibility that the wall part 40 breaks due to the air inside the wall part 40 is low even when winding the enclosing member 92 to the holding jig 120. The "enclosing-member winding step" of FIG. 6 shows such state.

Once the enclosing member 92 is wound to the holding jig 120, the holding jig 120 is pulled out of the enclosing member 92. The enclosing member 92 is still pinched in the hook-like portion of the loading jig 122. Once the holding jig 120 is pulled out, the tubular shaped portion of the double-folded sheet is held against the hook-like portion of the loading jig 122 (the "loading step" of FIG. 6 shows such state) and the enclosing member 92 is inserted thereinto using a rod 124. FIG. 5(C) shows such state.

The next step will be explained while again referring to FIG. 5. Once the enclosing member 92 is inserted into the double-folded sheet, heat is applied to the portion where the third weak seal portion 108 is to be formed, from the outside thereof. Thereby the third weak seal portion 108 is formed by fusion-bonding the adhesive agent applied to the inner surfaces of the sheet. FIG. 5(D) shows such state.

Once the third weak seal portion 108 is formed, the swallowing aid substance 90 is filled into the portion between the double-folded sheet that is not yet closed. Once the swallowing aid substance 90 is filled in, heat is applied from the outside to the portion where the inner strong seal section 102 is to be formed, for a predetermined time and at a predetermined pressure and temperature according to preset conditions for the inner strong seal section 102. Thereby, the inner surface of the sheet is fusion-bonded to form the inner strong seal section 102. FIG. 5(E) shows such state.

Once the inner strong seal section 102 is formed, a portion of the outlet chamber 80 is inserted into the cover section 88. FIG. 5 (F) shows such state. Thereby, the preparation 70 for oral administration of this example is completed.

Next, the procedure for taking out and ingesting the enclosing member 92 from the preparation 70 for oral administration of this example will be explained.

First, the caretaker etc. presses the swallowing-aid substance storage chamber 86 from the outside of the preparation 70 for oral administration to break the first weak seal portion 104 by means of the pressure from inside the swallowing-aid substance storage chamber 86. When the first weak seal portion 104 is broken, the swallowing aid substance 90 is pushed out into the intermediate chamber 84.
When the caretaker etc. further presses the swallowing-aid substance storage chamber 86 from the outside of the preparation 70 for oral administration, the second weak seal portion 106 is broken, the swallowing aid substance 90 is pushed out into the enclosing-member storage chamber 82. The swallowing aid substance 90 that was pushed out spreads inside of the enclosing-member storage chamber 82 and fills the surroundings of the enclosing member 92. From this time, the surface of the enclosing member 92 starts to melt in the swallowing aid substance 90.

After the swallowing aid substance 90 is pushed out into the enclosing-member storage chamber 82, the caretaker or patient or the like squeezes the preparation 70 for oral administration from the swallowing-aid substance storage chamber 86 towards the enclosing-member storage chamber 82. Thereby, the third weak seal portion 108 breaks as a result from the pressure applied to the swallowing aid substance 90. When the third weak seal portion 108 is broken, the swallowing aid substance 90 and the enclosing member 92 are pushed out into the outlet chamber 80. When the swallowing aid substance 90 and the enclosing member 92 are pushed out into the outlet chamber 80, the caretaker or patient or the like holds the aperture of the outlet chamber 80 to the mouth of the patient and squeezes the preparation 70 for oral administration from the swallowing-aid substance storage chamber 86 towards the enclosing-member storage chamber 82. Thereby the enclosing member 92, which is covered by the swallowing aid substance 90, enters the mouth of the patient. Note that the timing at which the aperture of the outlet chamber 80 is held against the mouth is not limited to the abovementioned one.

In the abovementioned way, the preparation 70 for oral administration of this example produces the following effects. The first effect is that it is possible to easily swallow a granular drug or another type of medicinal composition 50. The second effect is that tastes such as the bitter taste of drugs are suppressed. The third effect is that it is possible to suppress the scattering of the medicinal composition 50 in the mouth. The fourth effect is that concerns regarding the decomposition of the drug become unnecessary. The reason is that the medicinal composition 50 and the swallowing aid substance 90 (and consequently the moisture contained therein) are separated while the preparation 70 for oral administration is stored. The fifth effect is that it is possible to let patients with swallowing difficulties easily ingest solids of a size that such patients normally cannot easily swallow. The sixth effect is that it is possible to reduce the non-ingestible portion, in other words, the residual quantity of the medicinal composition 50 inside the preparation 70 for oral administration (in this example, it is possible to reduce the residual quantity to close to zero), in other words, that it is possible to ingest the drug of the predetermined quantity almost completely. The seventh effect is that it is possible to produce the preparation for oral administration quickly. The eighth effect is that the enclosing member 92 comes out smoothly. The ninth effect is that it is possible to easily ingest without water even for persons who have difficulties with the secretion of saliva. The tenth effect is that due to the use of the swallowing aid substance 90, it is possible to easily ingest the medicinal composition 50 reliably and without choking. The eleventh effect is that it is possible to ingest the medicinal composition reliably without touching the medicinal composition 50 or the swallowing aid substance 90 or the like, hygienically and without a scattering of the medicinal composition 50 to the outside.

The first effect will now be explained in detail. The enclosing member 92 enters the mouth of the patient while being covered by the swallowing aid substance 90. At this time, the surface of enclosing member 92 has melted. The enclosing member 92 can easily be swallowed even by a patient who has swallowing difficulties, because the enclosing member 92 is enclosed in the swallowing aid substance 90 and the surface of the enclosing member 92 has melted. Since the medicinal composition 50 is inside the enclosing member 92, the medicinal composition 50 is also swallowed with the swallowing of the enclosing member 92. Thereby it is possible to easily swallow the medicinal composition 50.

The second effect will now be explained in detail. As mentioned above, the enclosing member 92 enters the mouth of the patient while being covered by the swallowing aid substance 90. Thereby the medicinal composition 50, which is contained in the enclosing member 92, is doubly enclosed by the swallowing aid substance 90 and the enclosing member 92. Even if the medicinal composition 50 is a drug, the probability that the tongue of the patient senses the bitter taste thereof is low because the medicinal composition 50 is doubly enclosed. As a result, the bitter taste or other tastes of the drugs are suppressed.

The third effect will now be explained in detail. As mentioned above, the medicinal composition 50 is doubly enclosed by the swallowing aid substance 90 and the enclosing member 92. This reduces the probability that the medicinal composition 50 scatters in the mouth of the patient. As a result, it is possible to suppress the scattering of the medicinal composition 50 in the mouth.

The fourth effect will now be explained in detail. Drugs are often decomposed by moisture. In other words, normally, moisture causes drugs to generate decomposition products with unexpected side effects etc., and the decomposed drugs lose the pharmacological effect of their purpose. Compared to storing a drug in a liquid, the preparation 70 for oral administration of this example is stabilized because the liquid and the drug are separated. This is the reason why concerns regarding the stability of the drug are unnecessary when the preparation 70 for oral administration of this example is used.

The fifth effect will now be explained. As the swallowing aid substance 90 and the enclosing member 92 are ingested at the same time after the swallowing aid substance 90 is guided into the enclosing-member storage chamber 82, the preparation can pass the inside of the mouth smoothly because of the swallowing aid substance 90 covering the surface of the enclosing member 92. The effect achieved thereby is that it is possible to easily ingest a solid preparation of a size that cannot be easily swallowed normally.

The sixth effect will now be explained. When the swallowing aid substance 90 and the enclosing member 92 are pushed out from the aperture formed on the preparation 70 for oral administration, the medicinal composition 50 inside the enclosing member 92 will also be pushed out at the same time. This significantly reduces the residual quantity of the medicinal composition 50 in the enclosing-member storage chamber 82 as compared to cases where the medicinal composition 50 is not contained in the enclosing member 92. Moreover, the enclosing member 92 of this example is tightly sealed. Since the enclosing member 92 is tightly sealed, the probability of the medicinal composition 50 leaking from the enclosing member 92 is extremely low. Since this probability is extremely low, it is possible to reduce the residual quantity of the medicinal composition 50 inside the preparation 70 for oral administration (actually, it is possible to reduce the residual quantity close to zero).

The seventh effect will now be explained. The wall part 40 of the enclosing member 92 is formed by rolling one oblate sheet into a tubular shape. Therefore, the mouth of the enclosing member 92 is normally open. Since the mouth is normally open, the medicinal composition can easily be filled thereinto. Since the medicinal composition can easily be filled in, a fast production is possible. Moreover, when storing the enclosing member 92 in the drug container 72, the enclosing member 92 can easily be stored in the drug container 72. This is because the enclosing member 92 is bent. To that extent, a fast production is possible.

The eighth effect will now be explained. When the swallowing aid substance 90 tries to flow out of the drug container 72, the enclosing member 92 of this example receives the force from the swallowing aid substance 90 and is pushed out thereby. As a result, the enclosing member 92 comes out smoothly.

### <Example 3>

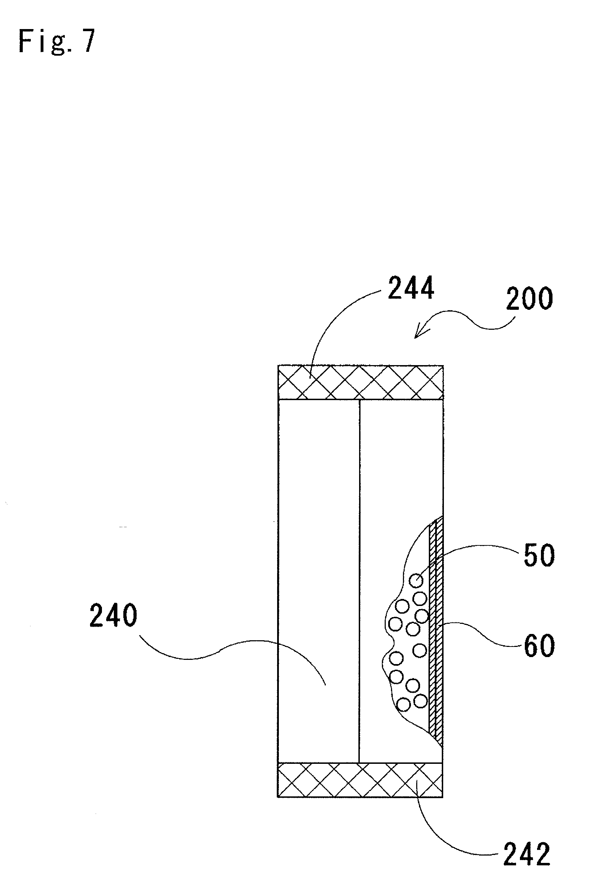

The preparation for oral administration of this example comprises only the enclosing member 200. FIG. 7 is a partial cross section of the enclosing member 200 of this example. The structure of the enclosing member 200 of this example will be explained on the basis of FIG. 7. The enclosing member 200 of this example contains a medicinal composition 50. The enclosing member 200 comprises a wall part 240, a bottom section 242 and a mouth section 244. In this example, the wall part 240 is made of two oblate (an example for an edible film) layers 60 that are overlaid. The layers are overlaid by rolling one oblate sheet into a tubular shape. In the example, the end of the oblate is not adhered except for the bottom section 242 and the mouth section 244. The bottom section 242 is formed by flatly compressing and fusion-bonding one end of the wall part 240. The mouth section 244 is formed by flatly compressing and fusion-bonding the end of the wall part 240 that is on the opposite side of the side where the bottom section 242 is disposed.

Next, the production method for the enclosing member 200 of this example will be explained. The production method for the enclosing member 200 of this example comprises a wall forming step, a bottom forming step, a medicinal composition filling step and a closing step. In the wall forming step, a tubular shaped wall part 240 is formed by rolling an oblate. In the bottom forming step, the bottom section 242 is formed by compressing one end of the oblate tube formed in the wall forming step and closing the compressed end by fusion-bonding. In the medicinal composition filling step, the medicinal composition is filled into the wall part 40. In the closing step, the mouth section 244 is formed by compressing the other end of the oblate tube that passed through the medicinal composition filling step and closing that other end by fusion-bonding.

Next, it will be explained how to ingest the enclosing member 200 of this example. The person intending to ingest the enclosing member 200 first places the enclosing member 200 into his or her mouth and immediately swallows the enclosing member 200 together with a sufficient quantity of water. The type of swallowing aid substance is not particularly limited, but a jelly is preferable.

Next, the effect of the enclosing member 200 of this example will be explained. The enclosing member 200 of this example is formed of an edible film (an oblate in this example) rolled into a tubular shape. Thereby the mouth of the edible film is open when the medicinal composition 50 is filled in. Since the mouth is open, the mouth of the auger filling machine (there are various shapes for the filling mouth and various filling methods for filling in the medicinal composition 50. It should be unnecessary to say that the use of an auger filling machine in this example is merely one in many examples.) can easily enter same. Since the mouth of the auger filling machine can easily enter, the efficiency of the medicinal composition filling step improves. Since the efficiency of medicinal composition filling step improves, the efficiency of the production of the enclosing member 200 improves.

Moreover, according to the enclosing member 200 of this example, the thickness of the wall part 240 can easily be changed by a change of the number of edible film layers. The thickness of the wall part 240 affects the elution speed of the medicinal composition 50 from the enclosing member 200; therefore being easily able to change the thickness of the wall part 240 means that the elution properties of the medicinal composition 50 being released from the enclosing member 200 can easily be controlled. As a result, it is possible to provide an enclosing member 200 which allows an easy control of the elution properties of the medicinal composition 50 which is released therefrom.

[Regarding leaks of the medicinal composition] Next, an explanation regarding leaks of the medicinal composition 50 in the enclosing member 200 of this example will be given. Leakage tests were performed according to the following procedure. First, a medicinal composition is filled into an enclosing member 200 of a diameter of 9 mm and a total length of 65 mm. Three enclosing members 200 filled with the same type of medicinal composition were produced. After the enclosing member 200 was produced, a 50-time powder leakage test and a 100-time powder leakage test, which will be explained later, were performed.

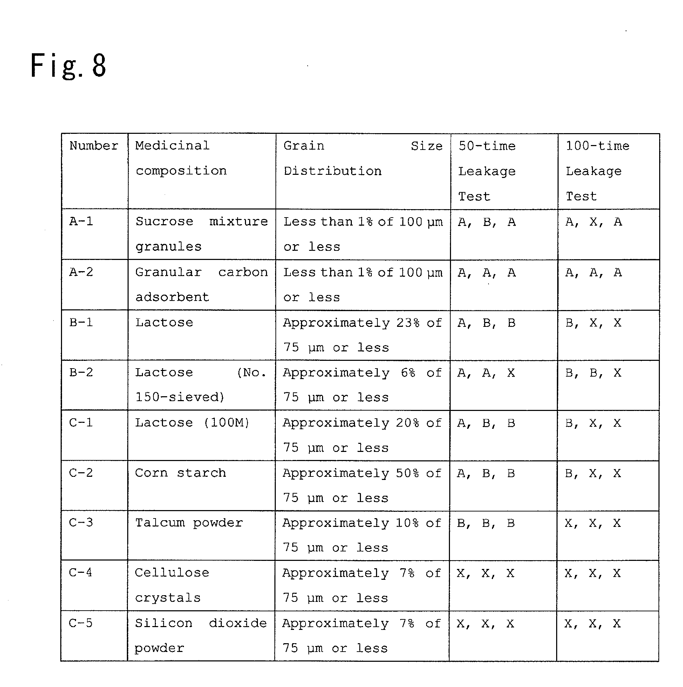

FIG. 8 shows the results of those leakage tests. The "Number" column shown in FIG. 8 indicates the lot numbers given to the enclosing members 200. The "Medicinal composition" column indicates the type of medicinal composition filled into the enclosing members 200. The "Grain Size Distribution" column indicates the grain size distribution of the medicinal compositions. "Grain size distribution" here case indicates the content of grains of a predetermined size. The "50-time Powder Leakage Test" column shows whether there was a leakage of the medicinal composition from the enclosing member 200 to the container when the enclosing member 200 was stored in a container (in this case, a container made by double-folding a soft resin and fusion-bonding the outer circumference thereof), and the person executing the test holds same in his or her hand and lightly hits same against a common office desk for 50 times. The existence of a leak was confirmed with the naked eye by the person executing the test. FIG. 8 shows the results of the 50-time powder leakage test for the abovementioned three enclosing members 200. The "100-time Powder Leakage Test" column shows whether there was a leakage of the medicinal composition from the enclosing member 200 to the container when, according to the same method as in the 50-time powder leakage test, the person executing the test lightly hit the container having the enclosing member 200 stored therein against a common office desk for 100 times. Same as for the 50-time powder leakage test, FIG. 8 shows the results of the 100-time powder leakage test for the abovementioned three enclosing members 200. The "A" marks in FIG. 8 indicate that no leakage could be confirmed with the naked eye. The "B" marks show that a very small leakage was confirmed with the naked eye. The "X" marks show that the medicinal composition leaked to a degree that cannot be described as "slightly." However, the "B" mark and "X" mark in the row where the "Number" column says "A-1" are due to the enclosing member 200 having been damaged. Said "B" mark and "X" mark are not due to a leakage from a gap between the oblate layer 60 of the wall part 240.

According to FIG. 8, no leakage of the medicinal composition occurs at all if the medicinal composition contains at least 99 weight percent of particles having a particle size of more than 100 µm, except for the case where the enclosing member 200 itself was damaged. That is to say, no leakage of the medicinal composition occurs at all if the medicinal composition contains less than 1 weight percent of particles having a particle size of 100 µm or less, except for the case where the enclosing member 200 itself was damaged. Leakages of the medicinal composition do not occur very much also in the case where the medicinal composition contains 10 weight percent or more of particles with a particle size of 75 µm or less.

### <Alternative examples>

The preparations for oral administration explained in the above examples are presented as examples to concretize the technical concept of this invention. Various changes may be applied to the preparations for oral administration explained in these examples within the scope of the technical concept of this invention.

For example, as the material for the enclosing members 20, 92 and 200, a variety of other materials conventionally known as edible film materials can be used apart from an oblate made of starch with a thickness of 10 µm as mentioned above. Those materials may be used on a standalone basis or as a combination of two or more types. As the material for forming the edible film, it is possible to use, for example, chitin, chitosan, casein, sodium caseinate, gelatin, collagen, soybean protein, egg white, starch, pectin, arabinoxylan, soybean polysaccharide, sodium alginate, carrageenan, agar-agar, glucomannan, galactomannan, xanthan gum, pullulan, zein, shellac, a guar gum degradation product, cellulose or another type of natural polymer, dextrin, etherified starch, esterified starch, hypromellose (HPMC; former Japanese pharmacopoeia name "hydroxyl propyl methyl cellulose"), hydroxypropyl cellulose (HPC), water-soluble hydroxyethyl cellulose (HEC), methyl cellulose (MC), carboxymethyl cellulose (CMC) or another type of semisynthetic polymer, polyvinyl alcohol (PVA), polyvinyl pyrrolidone (PVP), polyethylene glycol-polyvinyl alcohol copolymer, sodium polyacrylate, polyacrylamide (PAA), polyethylene oxide (PEO), polyethylene glycol (PEG), a silk protein degradation product, a casein degradation product or another type of synthetic polymer.

As the material of the enclosing members 20, 92 and 200, other components may be included in addition to the abovementioned variety of ingredients. Such "other components" include, for example, plasticizers, corrigents, flavoring agents, emulsifiers and coloring agents. A plasticizer is a component for improving flexibility and preventing the occurrence of cracks and breaks.

Further, the wall part 40 of the enclosing members 20 and 92, and the wall part 240 of the enclosing member 200 do not necessarily comprise the edible film layer 60 over their entire circumference. For example, the portion where edible films overlap to form the edible film layer 60 may be only a very small portion of the wall part 40 or 240. In this case, the wall part 40 or 240 may be formed into a tubular shape with the two ends of one edible film sheet overlapping only a little. However, if the wall part 40 or 240 is formed in such manner, it is preferable that the edible film overlaps in at least half of the entire wall part 40 or 240.

Moreover, the end of the edible film forming the wall part 40 may be adhered or not adhered to the wall part 40. In the case of the former, the outer circumference of the wall part 40 will become closed by the adhesion of the end of the edible film to the wall part 40. In the case of the latter, there will be a gap between the end of the edible film and the edible film on the inside thereof. This is the same regardless of whether the two ends of one edible film sheet are formed into a tubular shape while being overlapped only a little, whether one edible film sheet is formed into a tubular shape by being rolled in many layers, or whether a separate edible film was wound over one edible film sheet rolled into a tubular shape.

Further, regarding the enclosing members 20 and 92 which are wound into a curl shape, the end of the enclosing member 20 or 92 may be fixed or not fixed to another portion of the enclosing member 20 or 92. In the case of the former, the end of the enclosing member 20 or 92 is fixed to another portion of the enclosing member 20 or 92, and therefore the enclosing member 20 or 92 maintains the form of a curl shape.

Further, in the abovementioned examples 1 and 2, cases were explained where the medicinal composition 50 is a powdered or granular preparation and the swallowing aid substance 90 is a jelly; however, it is needless to say that the medicinal composition 50 and swallowing aid substance 90 applied to this invention are not limited thereto. For example, apart from a powdered or granular preparation, the medicinal composition 50 may be a pharmaceutical powder, crystals, a tablet, a capsule or a simple block. The swallowing aid substance 90 may be simply water, an aqueous solution, or may also be honey, custard cream, peanut spread, cheese spread, syrup or the like. The problem of what kind of substance to employ as the swallowing aid substance 90 is mainly solved on the basis of the nature of the medicinal composition 50. For example, if the medicinal composition 50 has an oil-soluble nature, it is preferable that the substance employed as the swallowing aid substance 90 contain oil and/or fat. The reason is that thereby the preparation is expected to smoothly pass the throat of the patient. Moreover, the item filled into the enclosing member 20 or 92 as the medicinal composition 50 is not limited to an item that is usually treated as a medical drug. For example, such item may be food.

Furthermore, in the abovementioned examples 1 and 2, the medicinal composition 50 was filled using an auger filling machine 154, but the device for filling in the medicinal composition 50 is not limited to the auger filling machine 154. For example, the filling machine for filling the medicinal composition 50 may be of a vacuum compression type, a piston type, a gauge type, a tapping type or another type. However, the auger filling machine 154 is preferable in that the auger filling machine 154 allows filling in the predetermined quantity of the medicinal composition 50 in a soft and easy manner, and also enables an easy control of the filling quantity. The concrete structures of these filling machines are known; thus a detailed explanation thereof will not be repeated here. Further, it should be unnecessary to say that these matters are the same in example 3.

Moreover, the orientation and form of the enclosing members 20 and 92 are not limited to those explained in the abovementioned examples 1 and 2. For example, the enclosing members 20 and 92 may be folded over lengthwise or widthwise. In this case, it is preferable that both ends of the folded enclosing member 92 of example 2 are oriented towards the swallowing-aid substance storage chamber 86. The reason is that in this way, there is a higher probability that the enclosing member 92 is pushed out together with the swallowing aid substance 90.

Further, in the abovementioned example 1, the structure and material of the package bag 22 are not limited to those mentioned above. For example, the weak seal 32 may be disposed in a position on the package bag 22 closer to the mouth than the sealing zipper 34. If the seal is disposed in a position on the package bag 22 closer to the mouth than the sealing zipper 34, the seal may also be a seal that is not weak but strongly fusion-bonded to a degree that the seal does not break even when being pulled from the outside of the package bag 22. To take out the enclosing member 20 in this case, it is also possible to tear apart a V-shaped notch provided on the package bag 22 and remove the seal portion. Further, apart from synthetic resin, the material of the package bag 22 may also be paper, aluminum film or the like. Moreover, a multilayer film or another type of composite material may be used as the material for the package bag 22.

Further, the number of spaces provided in one drug container 72 in the abovementioned example 2 may be only two, or may be three or more.

The preparation 70 for oral administration in the abovementioned example 2 is not limited to being formed by double-folding and adhering one sheet. For example, the preparation 70 for oral administration may be made by adhering two sheets to each other, or by adhering one tube at several positions.

Further, the abovementioned example 2 explained a preparation for oral administration provided with an outlet chamber 80, but the outlet chamber 80 does not necessarily have to be provided. If no outlet chamber 80 is provided, it is also possible to provide a notch on the enclosing-member storage chamber 82, apply shearing force to that notch to form an opening, and let the enclosing member 92 be ingested from that opening.

Further, in the abovementioned example 1, a mouth section 44 is provided on the enclosing member 20 which is rolled into a curl shape, but a mouth section 44 does not necessarily have to be provided. If a mouth section 44 is provided on the wall part 40 and there is no vent hole, it is possible that the wall part 40 breaks due to the air inside the enclosing member 92 when the enclosing member 92 is rolled into a curl shape. If the end of the edible film is not adhered to the wall part 40, the gaps of the edible film layers 60 forming the wall part 40 serve as a vent hole and the wall part 40 will not break. If the wall part 40 is adhered, a vent hole is necessary; therefore no mouth section 44 is provided. For the same reason, no mouth section 44 is provided on the enclosing member 92 rolled into a curl shape in example 2, but if the end of the edible film forming the wall part 40 is not adhered to the wall part 40, a mouth section 44 may be provided.

### Explanation of the Reference Numerals

- 10, 70:: Preparation for oral administration
- 20, 92, 200:: Enclosing member
- 22:: Package bag
- 30:: Package-bag bottom section
- 32:: Weak seal section
- 34:: Sealing zipper
- 40, 240:: Wall part
- 42, 242:: Bottom section
- 44, 244:: Mouth section
- 50: Medicinal composition
- 60:: Oblate layer
- 72:: Drug container
- 80:: Outlet chamber
- 82:: Enclosing-member storage chamber
- 84:: Intermediate chamber
- 86:: Swallowing-aid substance storage chamber
- 88:: Cover section
- 90:: Swallowing aid substance
- 100:: Circumferential strong seal portion
- 102:: Inner strong seal section
- 104:: First weak seal portion
- 106:: Second weak seal portion
- 108:: Third weak seal portion
- 120:: Holding jig
- 122:: Loading jig
- 124:: Rod
- 130:: Oblate
- 150:: Adsorption tube
- 152:: Edible film container
- 154:: Auger filling machine
- 160:: Predetermined aperture part

## Claims

1. A preparation for oral administration comprising an enclosing member made of edible film, wherein the aforementioned enclosing member contains a medicinal composition, the aforementioned enclosing member comprises a wall part, and the aforementioned wall part is formed by rolling the aforementioned edible film into a tubular shape.

2. The preparation for oral administration set out in claim 1, wherein the aforementioned wall part comprises a portion where the aforementioned edible film overlaps.

3. The preparation for oral administration set out in claim 2, wherein the aforementioned portion where the aforementioned edible film overlaps is formed by rolling one sheet of the aforementioned edible film.

4. The preparation for oral administration set out in claim 2, wherein the end of the aforementioned edible film is adhered to the aforementioned wall part.

5. The preparation for oral administration set out in claim 1, which is further equipped with a package bag, wherein the aforementioned enclosing member is stored in the aforementioned package bag.

6. The preparation for oral administration set out in claim 1, wherein the aforementioned enclosing member comprises a bottom section disposed on one end of the aforementioned wall part, and further comprises a mouth section which is disposed on the other end of the aforementioned wall part and closed.

7. The preparation for oral administration set out in claim 1, wherein the aforementioned preparation for oral administration is, in addition to the aforementioned enclosing member, further equipped with a drug container provided with a plurality of spaces, wherein the portions between two adjoining aforementioned spaces are closed, the aforementioned portions between two adjoining spaces open when force is applied from the outside of the aforementioned drug container, the aforementioned drug container is provided with a predetermined aperture part predetermined for forming an aperture which lets the outside of the drug container communicate with the aforementioned spaces, a swallowing aid substance is stored in a swallowing-aid substance storage chamber which is at least one of the aforementioned spaces, and the aforementioned enclosing member is stored in an enclosing-member storage chamber which is at least one of the aforementioned spaces.

8. The preparation for oral administration set out in claim 7, wherein the aforementioned enclosing member is stored in the aforementioned storage member storage chamber in a state where the aforementioned wall part is bent.

9. The preparation for oral administration set out in claim 8, wherein the state where the aforementioned wall part is bent is a state where the aforementioned wall part is bent to a curl shape.

10. The preparation for oral administration set out in claim 8, wherein the two ends of the aforementioned enclosing member are oriented towards the aforementioned swallowing-aid substance storage chamber.

11. A preparation for oral administration comprising an enclosing member made of edible film, wherein the aforementioned enclosing member contains a medicinal composition, the aforementioned enclosing member comprises a wall part, the aforementioned wall part is formed by rolling the aforementioned edible film into a tubular shape, the aforementioned wall part comprises a portion where the aforementioned edible film overlaps, and the aforementioned medicinal composition contains at least 99 weight percent of particles with a particle size of more than 100 micrometers.

12. A production method of a preparation for oral administration, wherein the aforementioned preparation for oral administration comprises an enclosing member made of edible film, the aforementioned enclosing member contains a medicinal composition, the aforementioned enclosing member comprises a wall part consisting of the aforementioned edible film; and wherein the aforementioned production method comprises a wall forming step in which the aforementioned wall part is formed by rolling the aforementioned edible film into a tubular shape, and a medicinal composition filling step in which the aforementioned medicinal composition is filled from one end of the aforementioned wall part into the space enclosed by the aforementioned wall part.

13. The production method of the preparation for oral administration set out in claim 12, wherein the aforementioned enclosing member comprises a bottom section disposed on one end of the aforementioned wall part, and further a mouth section which is disposed on the other end of the aforementioned wall part and closed; and wherein the aforementioned production method comprises a bottom forming step in which the aforementioned bottom section is formed by compressing and fusion-bonding one end of the aforementioned wall part, and further a closing step in which the aforementioned mouth section is closed by compressing and fusion-bonding the other end of the aforementioned wall part after the aforementioned medicinal composition filling step.
